# EUROPEAN PATENT SPECIFICATION

(11) **EP 4 444 167 B1**
(45) Date of publication and mention of the grant of the patent: **18.11.2026**
(21) Application number: 22829804.8
(22) Date of filing: 08.12.2022
(51) Int. Cl.: A61B 5/03, A61B 5/00, A61F 5/00, A61B 5/22

(54) **ANORECTAL MANOMETER**
ANOREKTALES MANOMETER
MANOMÈTRE ANORECTAL

(30) Priority: 08.12.2021 GB 202117707
(43) Date of publication of application: 16.10.2024
(73) Proprietor: Lucid Group Limited, Manchester Greater Manchester M15 6JJ (GB)
(72) Inventor: WILLIAMSON, Alistair, Manchester Greater Manchester M15 6JJ (GB); HODGIN, Andrew, Manchester Greater Manchester M15 6JJ (GB); STONE, Carl, Manchester Greater Manchester M15 6JJ (GB); PHAM, Nguyen, Manchester Greater Manchester M15 6JJ (GB); LEVER, James, Manchester Greater Manchester M15 6JJ (GB); WONG, Jan, Manchester Greater Manchester M15 6JJ (GB); HOLLIDAY, Thomas, Manchester Greater Manchester M15 6JJ (GB)
(74) Representative: Bartle Read
(86) International application number: PCT/GB2022/053141
(87) International publication number: WO 2023/105238

(56) References cited:
- EP-A1- 2 322 110
- US-A- 5 533 515
- US-A1- 2009 222 058
- LEE YEONG YEH ET AL: "High Resolution and High Definition Anorectal Manometry and Pressure Topography: Diagnostic Advance or a New Kid on the Block?", CURRENT GASTROENTEROLOGY REPORTS, SPRINGER US, NEW YORK, vol. 15, no. 12, 23 November 2013 (2013-11-23), pages 1 - 8, XP035359456, ISSN: 1522-8037, [retrieved on 20131123], DOI: 10.1007/S11894-013-0360-2

## Description

The present invention is concerned with anorectal manometers.

The term "manometry" refers in general to measurement of pressure in relation to the body. Often this is done using some form of insertable probe. Anorectal manometry is used in the assessment of disorders leading to faecal incontinence and constipation but may also be appropriate for patients presenting with other conditions and symptoms including rectal prolapse, perianal soreness, neurological or spinal injury or disease including spina bifida or stroke, and others. It involves measurement of pressure exerted upon a probe inserted through the anus and extending into the rectum.

It should be emphasised that while manometers are often used diagnostically, they may also be used therapeutically, for example in relation to exercises to be carried out by a patient or user. In this context the manometer may provide feedback about the user's performance of the exercises.

Anorectal manometers are used in a range of diagnostic tests and procedures. By way of example only, and without limitation, a diagnostic session might include:
- a resting pressure test;
- a squeeze test, in which the patient is instructed to squeeze with the anal sphincter and the maximal pressure achieved is measured;
- an endurance test, in which the patient is instructed to sustain pressure with the anal sphincter for a certain period (say 30 seconds) and the pressure profile over that time is measured;
- a cough-reflex test, involving measurement of rectal and anal pressure profiles;
- an attempted defecation or 'push' test, during which rectal and anal pressures may again be measured. A normal response involves an increase in rectal pressure coordinated with a relaxation of the anal sphincter. Some patients show a problematic increase in anal pressure, which can be diagnostically significant.

The process may also include providing a stimulus and assessing the patient's response to it. For this purpose the anorectal probe may carry a balloon, positioned during testing in the rectum, which is inflated to a variable degree after insertion.

The balloon can be used in a test of recto-anal reflex activity. Distension of the rectum is, in a healthy patient, normally followed by a transient increase of anal pressure followed by relaxation of the internal anal sphincter (the "recto-anal inhibitory reflex"). The balloon is used to produce the distension intended to evoke the reflex, and manometry is used to establish whether the expected response takes place.

The balloon may also be used in testing of rectal compliance, by observing variation of rectal pressure with balloon volume. It may also be used to make an estimation of rectal volume, or to determine whether the patient's rectal volume is abnormally large (a condition referred to as "megarectum"). These assessments may be made by introduction of a known volume of a pressure medium into the balloon, and observation of resultant pressure variation in the balloon. For this purpose the balloon's internal pressure needs to be sensed.

Other tests involve reporting of sensation by the patient. For example, as the balloon volume is increased measurements may be made of (a) the volume threshold for first sensation; (b) the volume threshold for a desire to defecate; and (c) the maximum tolerable volume. Still further known testing protocols include simulated defecation, and a balloon expulsion test.

Manometry systems currently in use are often of the water-perfused type in which a probe carries a set of water-filled tubes each leading to a sensor unit which monitors either water pressure or flow resistance. The tubes are deformed by the action of the body and the sensor unit thus receives information which can be used as an indication of pressures in the anus and rectum. Systems of this type suffer from several disadvantages. They are typically too large to be easily portable. Purchase cost of such systems is high. The probe itself is in many cases disposable, which is attractive from the point of view of infection prevention but adds to the expense of each procedure. Some systems need to undergo a time-consuming calibration process prior to use, which again adds to per-procedure costs. As a result such systems are, at least in the UK, typically found in specialist units. Referral rates for testing are low.

There are existing anorectal manometers which use sensors of other types.

US5533515A (John Coller et al) concerns a system for providing real-time measurements of pressure applied by the inner surface of a body lumen comprising an insertable probe provided at its tip with an inflatable balloon. The probe has a matrix of pressure transducer regions.

XP0353594556 (Lee Yeong Yeh et al) "High Resolution and High Definition Anorectal Manometry and Pressure Topography: Diagnostic Advance or a New Kid on the Block?" is about a form of anorectal manometer which is provided in some versions with an inflatable balloon toward its tip. In one version, the balloon is attachable to the probe through a removable male and female type connector.

US2014/0275841A1 (Borazjni et al) describes a probe insertable in a bodily lumen (the vagina in particular is referred to) having a single pressure chamber containing fixed and floating crystals employed in a technique of sonomicrometry.

GB2076293A (Steer) is concerned with a form of exercise device for sphincter muscles comprising a hollow elongated support member which carries or defines a hollow testing sleeve. The device is perfused with a working fluid and a single pressure transducer senses the changes in pressure of the working fluid created by exercise. It appears that only a single pressure measurement is available at any given instant.

GB666348 (Arnold) is another simple form of exercise device having an insertable element in which a tubular diaphragm defines an air space. Pressure in this air space is detected in order to record exertion of pressure against the diaphragm, but again only a single pressure value appears to be available at any given instant.

US5674238 (Sample et al) concerns a perineometer which indicates the pressure created by vaginal contractions. The device has a probe with a bulbous inflatable end and a pressure transducer in fluid communication with the probe. The transducer is external to the body being connected to the probe through a flexible tube.

WO02/34328A1 (Best Medical International BV) concerns a probe for treatment of urinary or faecal incontinence which has a cylinder which can be brought to a desired internal pressure by means of an air channel and valve. A pressure sensor is provided for determining the pressure in the cylinder. The location of the pressure sensor is not shown in the document. Here again, only a single pressure measurement is made at a given moment in time.

EP3095385A1 (THD S.p.A.) describes a manometric probe having a single chamber in flow communication with a pressure sensor which is not part of the probe itself.

EP0219410 (Duhem) discloses a device for use in rehabilitation of urinary or anal incontinence, having a fluid filled chamber connected to a separate pressure sensing device.

DE3221115A1 (Maier) discloses a device for measuring activity of sphincter muscles having a probe insertable in a bodily orifice and comprising a tubular inner body connectable to an air source and a pressure gauge via a connecting line.

FR2970379A1 (Guerineau) concerns an anal or vaginal probe having a watertight chamber with displaceable sides. After insertion of the probe, liquid is injected into the watertight chamber to bring it into contact with the bodily muscles.

US5924984 (Rao) concerns an anorectal probe which uses transducers mounted on the probe itself to sense pressure, these being said to be of a "..variety commonly used for monitoring muscular activity", although the precise nature of the transducers is not specified.

US2012/0265044A1 (Broens) describes a probe provided on its exterior with electrical transducers serving as pressure sensors.

GB2446545 (Anatasol LLC) concerns a perineometer having a "transducer sensor body" formed by a textile fabric sleeve including a layer of polymeric piezoelectric material having metallized coating layers.

US2017/0128012A1 (Parks et al) concerns an extended balloon assembly for a catheter used in anorectal manometry, in which the balloon contains a through-going tube made in three parts. The catheter itself is to be inserted into this tube and is able to sense pressure in the balloon. No provision appears to be made to prevent contamination of the catheter during use.

Prevention of transmission of pathogens is an important issue in systems where the probe itself is intended to be re-used. It is vital to guard against transfer of pathogens from one patient to another by this route. There are existing anal probes that are provided with a replaceable sheath interposed between the patient's body and the probe itself. EP1996281 (Nephro - Urology Clinical Trials Limited) describes one such device, although it is described as a "wearable" device, which would presumably be intended for a single user, and suggests that the sheath would be replaced when discoloured through use. The sheath in question is also provided with electrodes formed of carbon-loaded silicone rubber, used to provide electrical stimulation. One important objective in infection control is to provide a device with a clean and simple exterior, without features likely to harbour pathogens.

It is necessary that a sheath to prevent transfer of pathogens should not impair the function of the manometer in sensing pressure. Where an inflatable balloon is to be provided, this needs to be done whilst still providing a barrier between the probe and external contaminants.

The functionality of the inflatable balloon also needs to be provided.

The invention is defined by the appended claims.

According to a first aspect of the present invention, there is a sheath for use with an anorectal manometer of the type comprising a probe comprising a proximal portion and a distal tip portion, the probe being insertable through an anus so that the distal tip portion extends into a rectum, and the probe being provided with a plurality of proximal pressure sensors responsive to pressures applied to the proximal portion of the probe,
the sheath being formed to removably receive the probe and to provide a continuous covering extending from the proximal portion to the distal tip portion of the probe, and the sheath comprising a proximal stem portion for receiving at least the proximal portion of the probe, a distal balloon portion, and an inflation conduit communicating with the balloon portion's interior, enabling inflation of the balloon portion with a pressure medium introduced through the inflation conduit whilst the balloon portion is disposed in the rectum,
wherein the sheath comprises flexible membranous material so that pressure applied to the stem portion of the sheath is transmitted through the sheath to the proximal portion of the probe, to be sensed by the pressure sensors, and
wherein the sheath comprises means for forming a seal against flow of the pressure medium from the balloon portion into the stem portion of the sheath.

Preferably the pressure transducers are solid-state devices. They may be piezoelectric devices. The substrate may be a circuit board. Preferably the flexible layer comprises elastomeric material. In a preferred embodiment, a unitary flexible layer forms multiple pressure cells. The flexible layer may have a shape which provides, for each pressure cell, an upstanding peripheral wall supporting a top. The flexible layer may be sealingly secured to the substrate. The substrate and the flexible layer are preferably disposed within a flexible cover arranged to transmit pressure to the flexible layer. This flexible cover may be a separate component, be applied to the flexible layer as an overmould or may be integral with the flexible layer.

The manometer may comprise at least two or at least three pressure cells spaced from one another along the probe's length.

In a preferred embodiment the probe has on its outer surface a channel configured to receive a tube extending at least part way from the probe's distal end to its proximal end, the tube being for inflation of a balloon coupled to the distal end of the probe.

The manometer of the present invention may be a self-contained hand-held unit requiring no physical connection to any other equipment during manometry. It may be provided with a wireless transceiver for sending manometry data to a digital processing device.

The manometer may be provided with a heating element for heating the probe to approximate body temperature. In this way spurious changes of measured pressure caused by changes of temperature in the pressure cells due to warming of the probe by the body can be avoided. The heating element may be controlled in a feedback loop based on measured probe temperature.

The flexible layer may be formed by overmoulding over the substrate. Preferably the flexible layer engages with the substrate via interlock formations, which resist movement of the flexible layer with respect to the substrate. The interlocks may comprise features of shape comprising any of recesses, projections, channels, trenches, shoulders or abutments. The interlocks are configured to resist rotational and/or longitudinal movement of the flexible layer with respect to the substrate. They do not interfere with flexure of the flexible layer needed to transmit pressure to the pressure cells.

Preferably the flexible skin comprises a pair of flat sheets seamed to one another along their peripheries. The flexible skin may comprise a relatively narrow portion to receive a probe part of the manometer, and a wider portion to receive and at least partly cover a body part of the manometer.

Note that while the invention is especially applicable to anorectal manometers, some aspects of the invention may equally well be applied to manometers for other bodily lumens. These may include vaginal manometers.

Specific embodiments will now be described, by way of example only, with reference to the accompanying drawings, in which:-
Figures 1 and 2 show the exterior of an anorectal manometer;
Figure 3 is an exploded view of the main components of the manometer;
Figure 4 is a side elevation of the manometer;
Figure 5 is a section through the manometer in a longitudinal plane;
Figure 6 is a section through a probe part of the manometer, somewhat simplified in that it omits a body part of the device;
Figure 7 depicts a sheath disposed upon the manometer;
Figure 8 depicts component parts of the sheath;
Figure 9 depicts a sheath embodying the present invention disposed upon the manometer;
Figure 10 corresponds to Figure 9 but is a sectional view;
Figure 11 depicts component parts of the sheath of Figures 9 and 10;
Figure 12 is a side view of another anorectal manometer;
Figure 13 is a sectional view through part of the manometer of Figure 12, and of a sheath carried on it;
Figure 14 depicts a collar of the sheath depicted in Figure 12, the collar being viewed from one side;
Figure 15 is a section through the collar of Figure 14, carried on the manometer;
Figure 16 is a section through the manometer carrying a further version of the sheath;
Figure 17 is a side view of still another anorectal manometer;
Figure 18 is a cross section through a probe part of the manometer of Figure 17;
Figure 19 is an exploded view of the manometer of Figure 17;
Figure 20 is a section through the probe part of the manometer of Figure 17 in an axial plane;
Figure 21 is a further sectional view through the probe part of the manometer of Figure 17 in an axial plane;
Figure 22 depicts a circuit board assembly of the manometer of Figure 17;
Figure 23 is a cross section through a probe part of the manometer of Figure 17 and a collar carried upon it, taken in the plane denoted H-H in Figure 25;
Figure 24 is a further cross section through a probe part of the manometer of Figure 17 and a collar carried upon it, taken in the plane denoted G-G in Figure 25;
Figure 25 is a section in an axial plane through the manometer of Figure 17 carrying a sheath embodying the present invention;
Figure 26 is a side view of the manometer of Figure 17 carrying a sheath embodying the present invention;
Figure 27 depicts a collar of the sheath;
Figure 28 represents a stage in the process of applying the sheath to the manometer of Figure 17;
Figure 29 depicts stages in the process of testing the manometer of Figure 17;
Figure 30 depicts a workstation carrying the manometer of Figure 17; and
Figure 31 is a section through the anorectal region of a patient showing the manometer of Figure 17 in use.

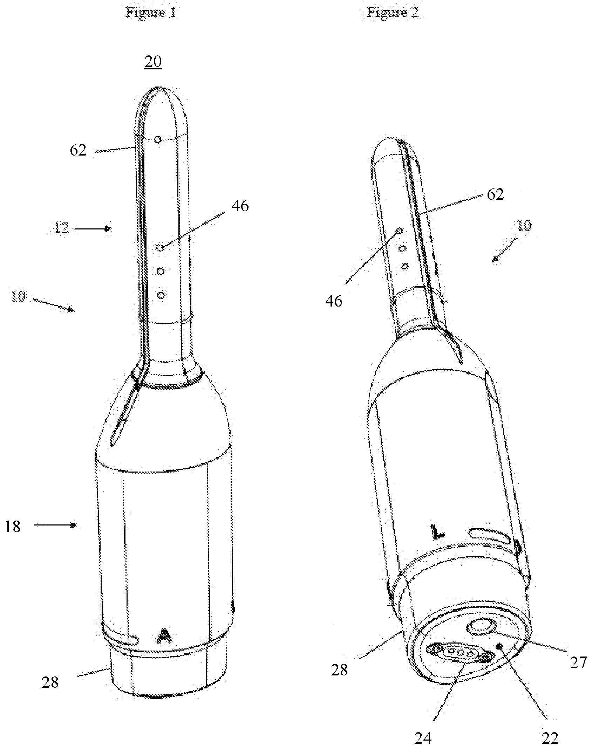

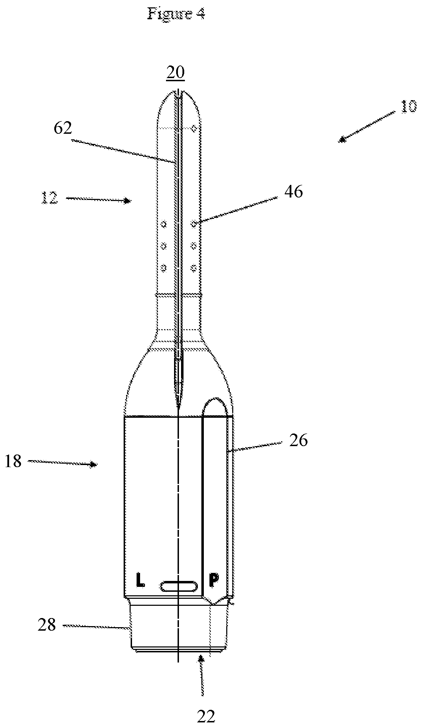

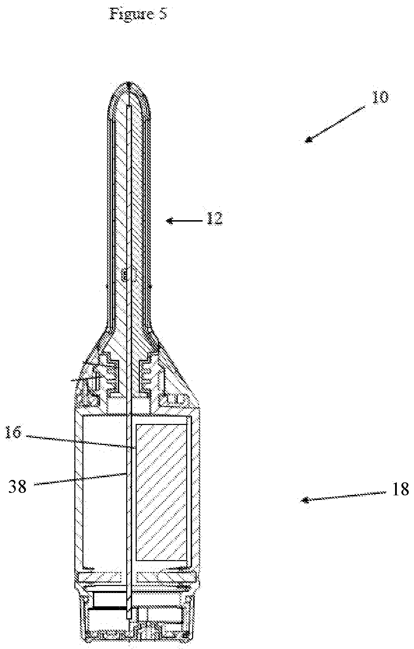

The manometer 10 represented in Figures 1 to 6 is a compact, hand-held unit whose uses include, but are not necessarily limited to, the type of clinical testing processes and therapies described above. It comprises a probe 12 which is insertable through a human anus and is long enough to extend through the anal canal into the rectum. Pressure sensors are configured to detect pressures applied to the probe 12. Data from the sensors is output, in the present embodiment, through a wireless or wired interface to a separately-formed processing and display unit, which may be in the form of a tablet computer. The manometer 10 in this example is powered by an on-board rechargeable battery 16 and is self-contained, in the sense that it needs no connection to an electrical power supply or to any source of pressurised fluid during use. It is capable of being reused a large number of times, and of resisting contamination of the manometer by pathogens, and their transmission from one patient to another.

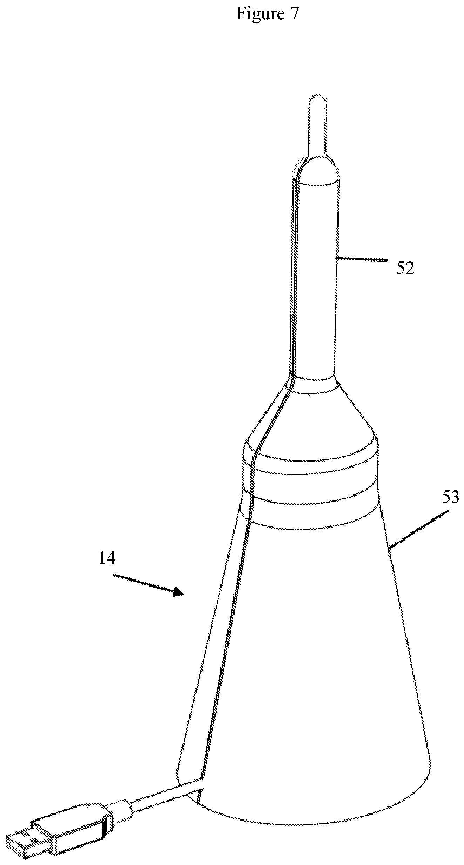

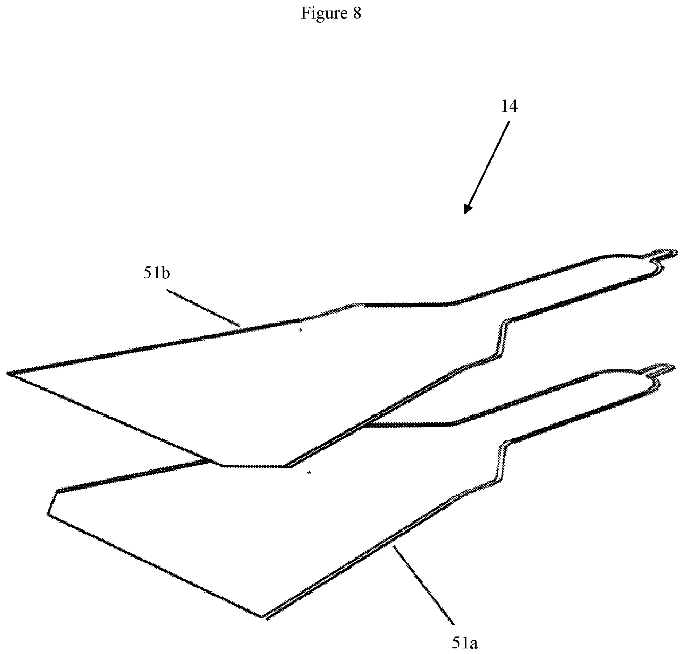

A single-use sheath 14 (see Figures 7 and 8) covers the manometer 10 in use and so prevents it from being contaminated by pathogens or other foreign matter, providing an important safeguard against transmission of infection from one patient to another.

The construction of the manometer 10 will now be described in more detail, although it is important to emphasise and to understand that much of the constructional detail could be quite different in another embodiment.

Outwardly (see for example Figures 1 and 2), the manometer 10 has an enlarged body 18 from which the probe 12 projects. The end of the probe inserted first will be referred to herein as the distal end 20. The end of the manometer opposite the distal end 20 will be referred to as the proximal end 22. The terms "proximal" and "distal" are to be correspondingly construed throughout.

In the present embodiment the proximal end 22 of the body 18 is formed by an end face provided with a first charging connector 24 and an on/off switch 27. The proximal end of the body 18 is shaped to seat in a charging station, not seen in Figures 1 to 6.

The body 18 carries markings to assist a user, such as a clinician, in achieving the required rotational alignment with respect to a patient's body when the probe 12 is inserted anally. In the present embodiment these take the form of the letters "A", "L", "P" and "R", standing respectively for "anterior", "left", "posterior" and "right", spaced at suitable intervals around the circumference of the device. The user will hold the device with the "A" facing forwards and the "R" to the right-hand side, in use. A groove 26 is formed in the body 18 to give the user tactile confirmation of the proper alignment of the manometer 10 in the user's hand. In the present embodiment this groove 26 runs up the posterior portion of the body 18, so that the user's thumb can naturally sit in it. Other features of shape could be used to give the user this tactile information. A record switch (not shown in Figures 1 to 6) may be disposed in the groove 26 and therefore easily operable using the thumb.

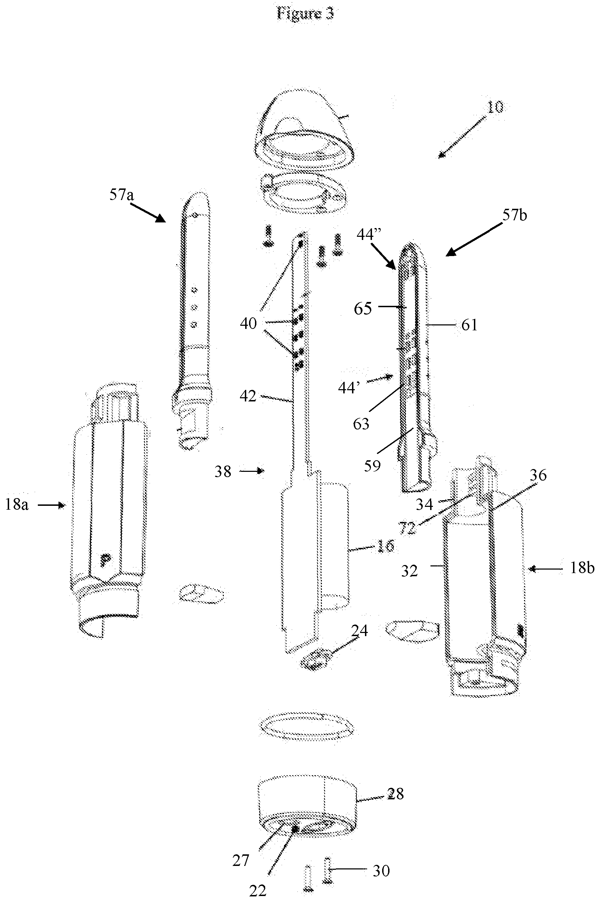

The body 18 comprises a pair of roughly semi-cylindrical body halves 18a, 18b whose proximal ends are covered and coupled by a cup-shaped end cap 28 (see Figure 3 in particular) which forms the proximal end face 22. Screws 30 passed through the end face 22 secure the charging connector 24 to it. The body halves 18a, 18b are assembled to one another in the finished product, meeting through abutment faces 32 (refer again to Figure 3). Alignment of one with respect to the other is achieved through complementary recesses 34 and tongues 36 formed on the abutment faces.

The present embodiment uses a single circuit board assembly 38 to serve several functions:
- it carries the battery 16, which is housed in the body 18;
- it carries transducers 40 used for pressure measurement;
- it carries battery management circuitry; and
- it carries the electronics required for receiving the sensor's outputs and transmitting sensor data wirelessly, or by wire, to an external unit.

The probe 12 comprises a pair of roughly semi-cylindrical probe parts 57a, 57b assembled to one another, each comprising a relatively rigid inner member 59 provided with a flexible outer layer 61. These may be moulded plastics components, the material of the flexible outer layer 61 being an elastomer layer over-moulded on the more rigid inner member 59. The inner member 59 has a set of cavities 63 each disposed to lie over a respective transducer 40 in the assembled device. It has a flat inner sealing face 65 which abuts and seals against the circuit board assembly 38. Each cavity 63 is open toward the probe's surface so that the cavities form respective sealed pressure cells 44 around each transducer 40.

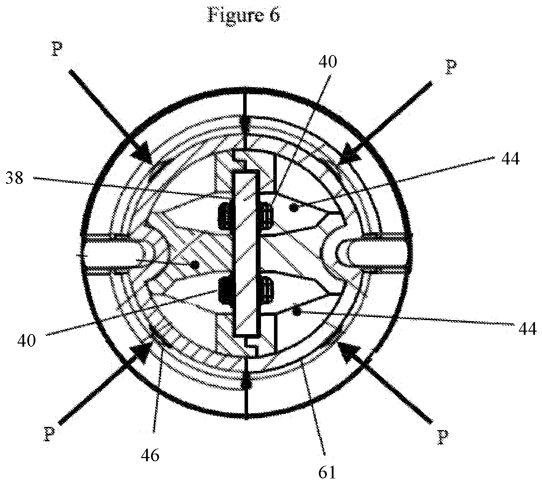

The pressure-sensing transducers 40 are mounted on a narrowed distal portion 42 of the circuit board assembly 38, each of them being arranged to sense pressure in a respective pressure cell 44. The pressure cells 44 are variable in volume. Application of pressure to a certain region of the outer layer 61 of the probe deforms that layer and causes a reduction in the volume of the pressure cell 44. Because the pressure cells 44 are sealed, this deformation produces a corresponding pressure increase in the cell, which is detectable by the transducer 40. It is in this way that pressures applied to the probe 12 are detected and measured. The pressure transducers 40 respond to local pressures exerted on respective the probe 12 along radial directions. Figure 6 includes arrows P representing pressures applied along different radial directions to each of four pressure cells 44. In this particular embodiment raised pips 46 are formed on outer layer 61 over each of the cells. These may be dispensed with in other embodiments. The action of the pressures P is to deform the outer layer 61 inwardly somewhat, thereby compressing pressure medium in the pressure cells 44 and raising pressures exerted on the transducers 40, causing them to vary their output signal in a manner representative of the applied pressure.

Note that the transducers 40 do not need to face along the direction that the applied pressures act. That is, they do not need to face radially. As a result, in the present embodiment, pressures can be sensed along four different radial directions using transducers placed on just two sides of the circuit board assembly 38, which makes construction and assembly of the probe 12 straightforward.

The transducers 40 may take any suitable form consistent with their function of sensing pressure in the cells 44 and outputting an electrical signal representative of that pressure. Micro-Electrical-Mechanical Devices (MEMS) are suitable and used in the present embodiment. More specifically, the transducers 40 used in the present embodiment are commercially available piezo-electric devices of this type.

The pressure cells 44 are filled in the present embodiment with air. In other embodiments it is possible that a different fluid would be used, such as a liquid or a different gas.

In the present embodiment the pressure cells 44 are arranged in groups (longitudinal rows) at regular circumferential intervals around the probe 12, specifically at ninety-degree intervals. A first group, indicated at 44' in Figure 3, is disposed along a proximal portion of the probe 12 to sense pressure exerted by the anal sphincter. A rectal pressure cell, indicated at 44" in Figure 3, is disposed in a distal portion closer to the distal end 20 of the probe 12 and serves to detect rectal pressure. When the manometer 10 is used with a sheath 14 providing a balloon portion 50, the rectal pressure cell 44" is able to sense gas or liquid pressure in the balloon portion 50, when the balloon is inflated, or to sense pressure exerted by the tissue of the rectum, when the balloon is not in inflated. A further pressure sensor is provided to sense ambient (barometric) pressure. Its output may be used to compensate for variations in the output signals of the transducers 40 due to variation of the ambient pressure.

As mentioned above, the manometer 10 is intended to be protected from contact with the patient and from contamination with pathogens or other foreign matter by means of a sheath placed over it prior to use. The sheath is to be disposed of and replaced after each use, thereby preventing transfer of pathogens from one patient to another.

A first example 14 of the sheath, lacking the balloon which is a feature of other embodiments of the invention, is represented in Figures 7 and 8, and comprises a body comprising a flexible impermeable elastomer skin shaped as a sleeve with a closed distal end, to be placed over the manometer 10. In the present embodiment the sheath 14 comprises a pair of shaped flat panels 51a, b joined to one another about their peripheries. This joining may be carried out by heat sealing. Quite different modes of manufacture could however be adopted in other embodiments.

The shape of the sheath provides a stem portion 52 sized to fit tidily upon the probe 12, and an enlarged portion 53 to fit over the manometer's body 18. The enlarged portion is in the present embodiment flared toward its proximal end to ease removal after use.

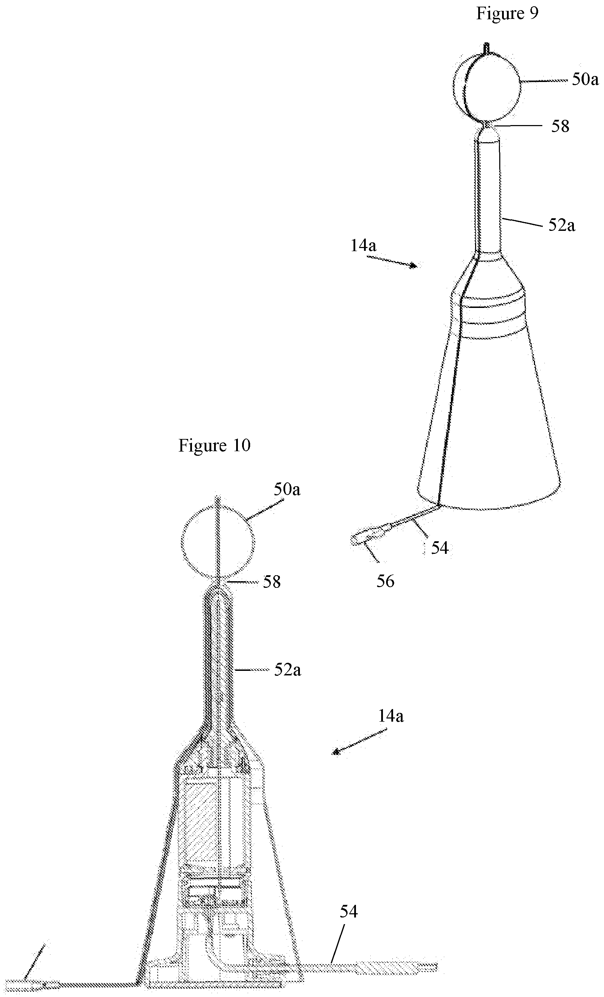

Figures 9, 10 and 11 represent a further embodiment 14a of the sheath, which is formed similarly to the earlier embodiment but differs from it in incorporating a balloon portion 50a at its distal end. The balloon portion 50a is inflatable within the patient's rectum in use by means of a conduit, more specifically in this embodiment a tube 54 passed through the interior of the sheath 14a to terminate in the balloon portion 50a. A fitting 56 on the proximal end of the tube 54 enables it to be connected to a syringe to inflate the balloon portion 50a. A constricted neck region 58 lies between the sheath's stem portion 52a and the balloon portion 50a, and the tube 54 passes through this neck region 58. But note that around the tube 54, the neck region 58 forms a seal against passage of pressure medium from the balloon portion 50a to the stem portion 52a of the sheath. That is, due to the seal in the neck region 58, the interior of the balloon portion 50 does not communicate with the interior of the stem portion 52a. Consequently, the balloon portion 50a is able to be inflated without escape of the pressure medium into the stem portion 52a.

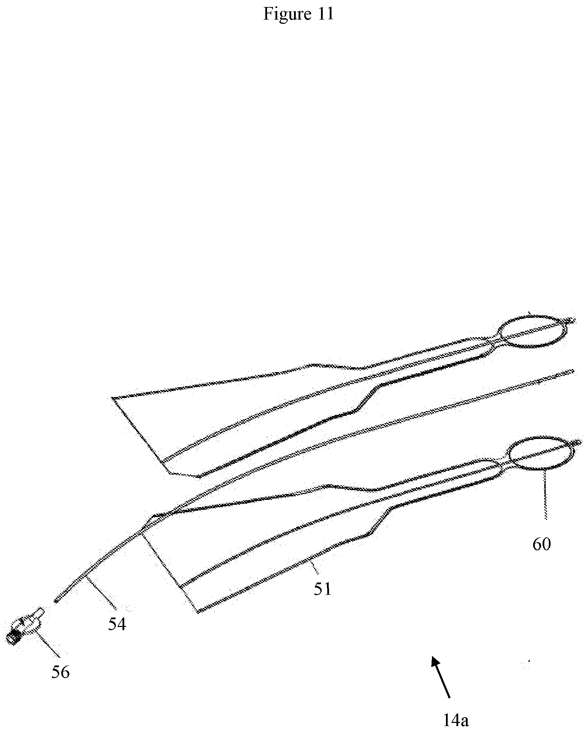

In the present embodiment the balloon portion 50a is formed by enlarged regions 60 at the distal ends of the flat panels 51 forming the sheath (see Figure 11). The enlarged regions 60 are joined to one another around their peripheries forming an internal space which is sealed save for the passage into it provided by the tube 54. They are circular in the present embodiment but other shapes could be used.

Referring to Figures 1, 2 and 4, the manometer 10 can be seen to have an elongate recess 62 in its outer surface extending the length of the probe 12 and along part of the length of the body 18. The tube 54 can be located in this recess in use so that it does not undesirably alter the outward shape of the probe 12, which might cause discomfort and/or affect measured pressures.

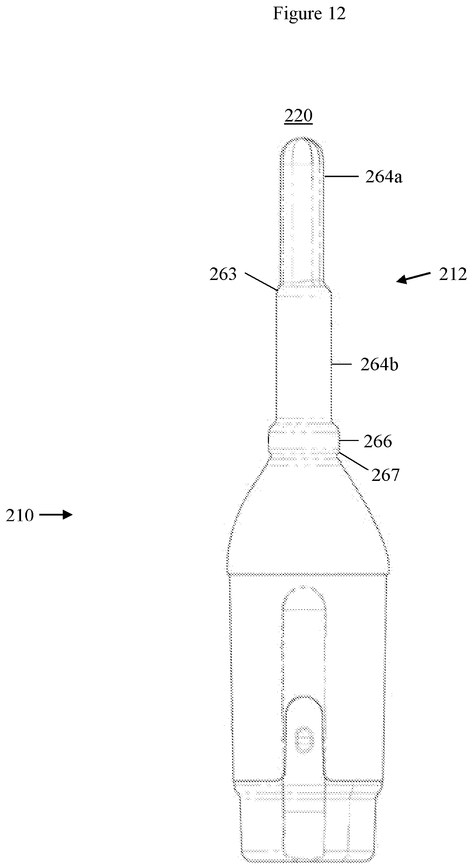

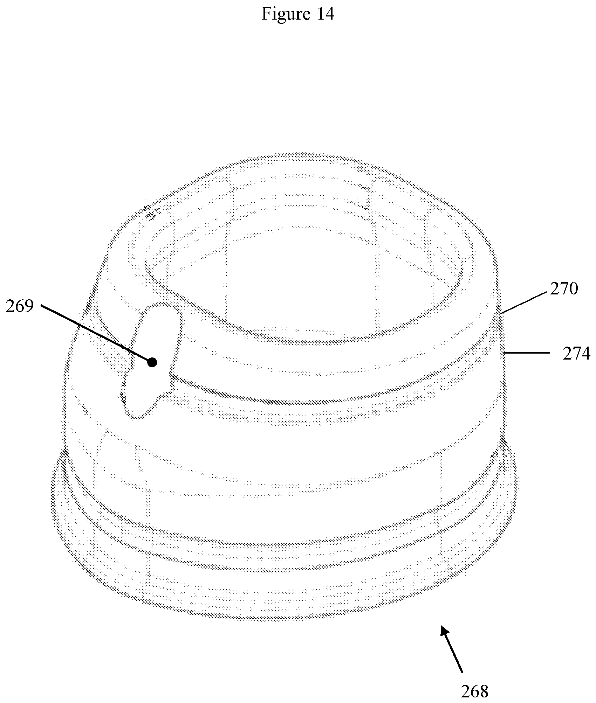

Figures 12 to 15 depict a second manometer 210. The internal construction and operation of the second manometer 210 are generally similar to those of the first embodiment and will not be described again here in detail. The exterior of probe 212, however, has some points of distinction from the earlier embodiment. Part-way along probe 212 is a location feature 263 which in the present embodiment comprises a shoulder forming a transition from a narrowed, distal, tip portion 264a of the probe 212 to a slightly broader proximal portion 264b of the probe 212. The location feature 263 serves to longitudinally locate sheath 214 with respect to the probe 212. At or toward the proximal end of the probe is a sealing feature 266 having an increased lateral dimension, and a circumferential groove 267.

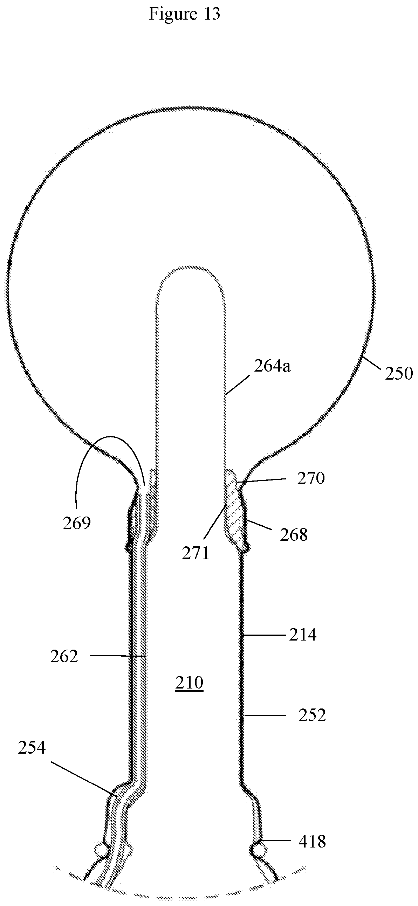

The sheath 214 depicted in Figures 13, 14 and 15 comprises a collar 268 which serves multiple functions, in this embodiment. In cooperation with the location feature 263 of the probe 212, the collar 268 provides positive longitudinal location of the sheath 214, and especially it locates its balloon portion 250 such that it contains tip portion 264a of the probe 212. During assembly, the collar 268 is advanced in the proximal direction until it engages the location feature 263, after which it can be advanced no further.

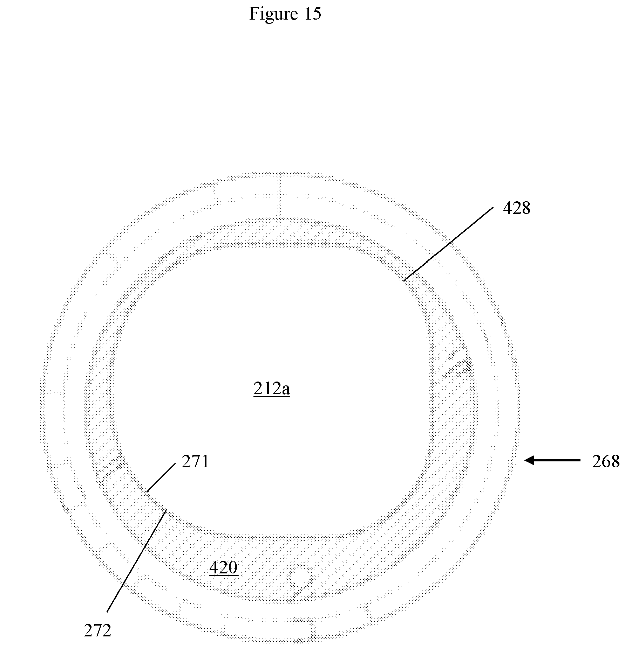

The collar 268 serves to mount tube 254 through which the balloon portion 250 is inflated and deflated. Tube 254 is received by the collar 268 and communicates with the balloon portion's interior via a passage through the collar 268 leading to an aperture 269. A circumferential groove 270 in the collar 268 prevents the sheath 214 from blocking the evacuation of gas or air from the balloon portion 250 by being sucked against the collar's surface. The collar 268 cooperates with the probe 212 to locate the tube 254 rotationally, aligning it with elongate recess 262 extending along the proximal portion 264b of the probe 212 to receive the tube 254. This rotational location is achieved, as can be appreciated from Figure 15, by virtue of the formation of the collar 268 with an asymmetric internal profile 271, and by providing the tip portion 264a of the probe 212 with a complementary asymmetric external profile 272. In this embodiment, these profiles have a rotational symmetry of order one, so that the collar 268 can only be advanced onto the tip portion 264a when in the desired rotational location, in which the tube 54 aligns with the recess 262.

The collar 268 provides in use a seal between the stem portion 252 of the sheath and its balloon portion 250. In the present embodiment, the conformable skin of the sheath 214 is bonded to a circumferential portion 274 of the collar 268. Without the presence of the probe 212, the interior of the balloon part 250 communicates with the interior of the stem portion 252 through the collar 268 itself. But when the sheath 214 is mounted on the manometer 210 as in Figures 13 and 15, the internal profile 271 of the collar 268 seals against the exterior of the probe 212. Hence pressure medium (air or gas) is able to enter or leave the balloon portion 250 only through the tube 254, and the balloon portion 250 is able to be inflated and deflated without affecting pressure in the stem portion 252, and so without affecting measurement of pressure applied through the stem portion 252 of the sheath to the proximal portion 264b of the probe 212.

As the distal tip portion 264a is within the balloon portion 250, its rectal pressure cell 44" (see Figure 3) responds to pressure of the pressure medium in the balloon portion 250 when the balloon portion 250 is inflated. When the balloon portion 250 is not inflated, this sensor responds to applied rectal pressure, without impairment by the flexible membranous material forming the balloon portion 250.

An O ring or other type of elastic band is received in the circumferential grove 267 to secure the stem portion 252 of the sheath 214 in place.

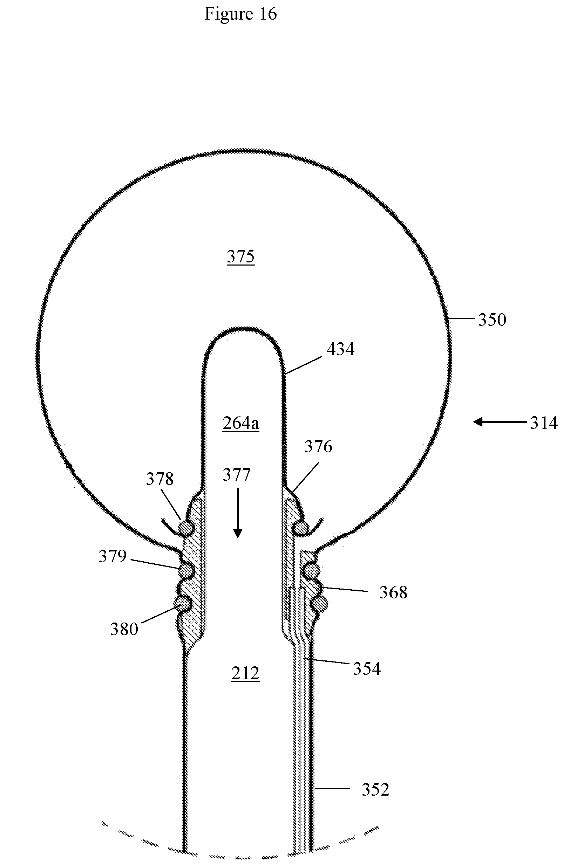

Figure 16 depicts an alternative form of sheath 314 for use with the manometer 210. This version again comprises a collar 368, but in this instance it is not necessary for the collar 368 to seal against the probe 212. Instead, an internal chamber 375 is formed within balloon portion 350 between the balloon portion 350 and a closure 376. The internal chamber is closed save for a passage leading to it through tube 354. The closure 376 seals passage 377 through the collar 368, whilst permitting the tip portion 264a of the probe 212 to be inserted through the collar 368, to lie within the balloon portion 350. The closure 376 comprises in this embodiment flexible membranous material (which may be the same type of material used to form the balloon portion 350 and stem portion 352 of the sheath). Consequently, while the closure 376 lies over the tip portion 264a of the probe 212, it does not impair the function of the rectal sensor, which is thus able to sense pressure in the balloon portion 350. The closure 376 meets and seals against the collar 368. This may be achieved by bonding the closure 376 to the collar 368, but in the present embodiment it is instead achieved by means of an O ring tension band 378 which traps the periphery of the closure 376 in a circumferential groove of the collar 368. Likewise, a neck portion of the sheath 314 is secured and sealed against the collar 368 within by means of a pair of O ring tension bands 379, 380 received in respective circumferential grooves of the collar 368.

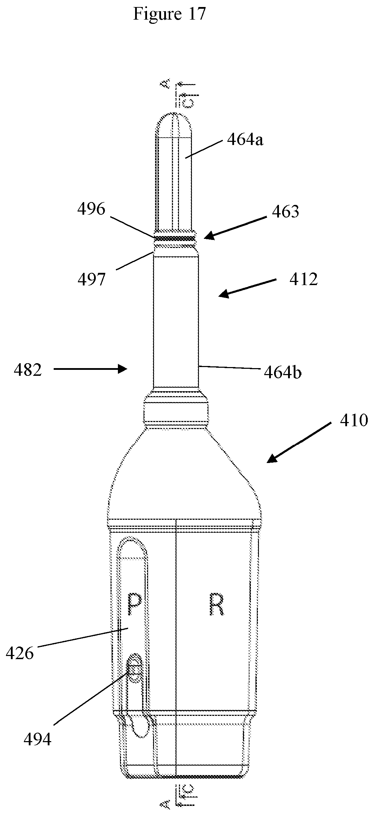

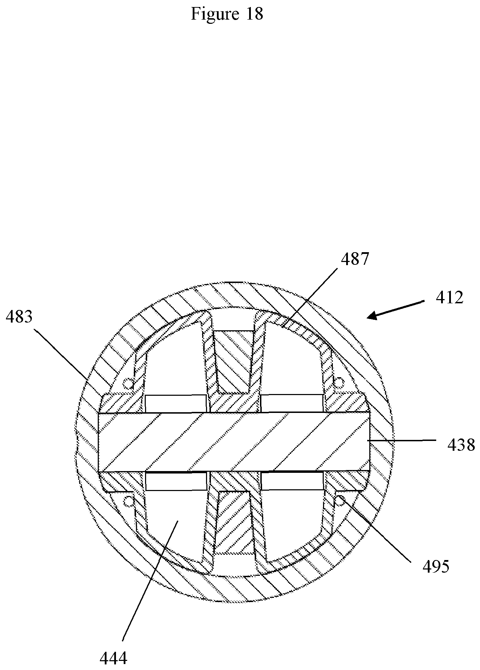

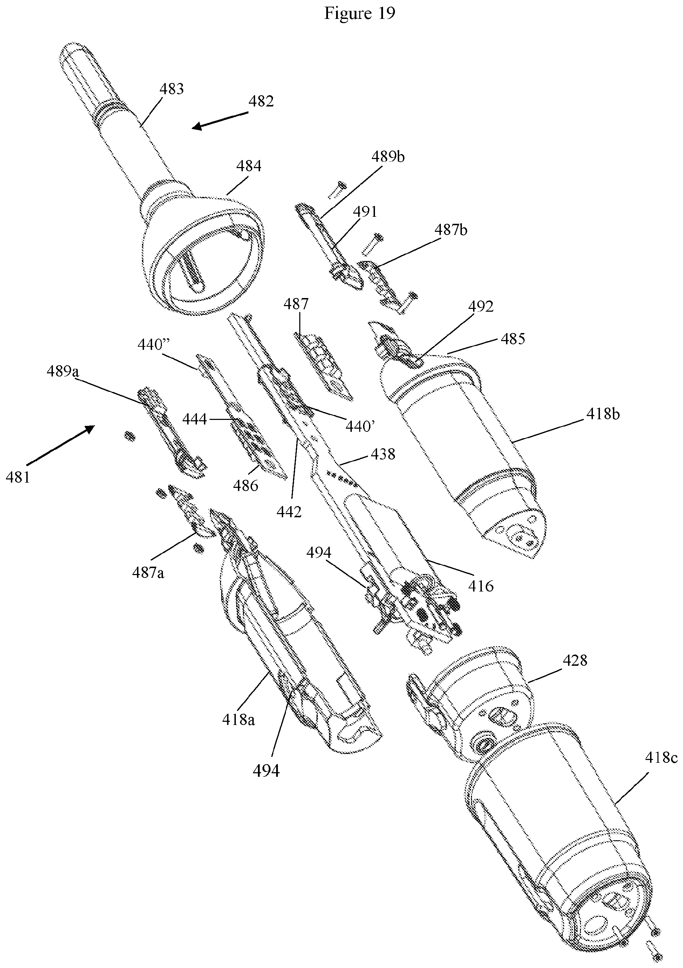

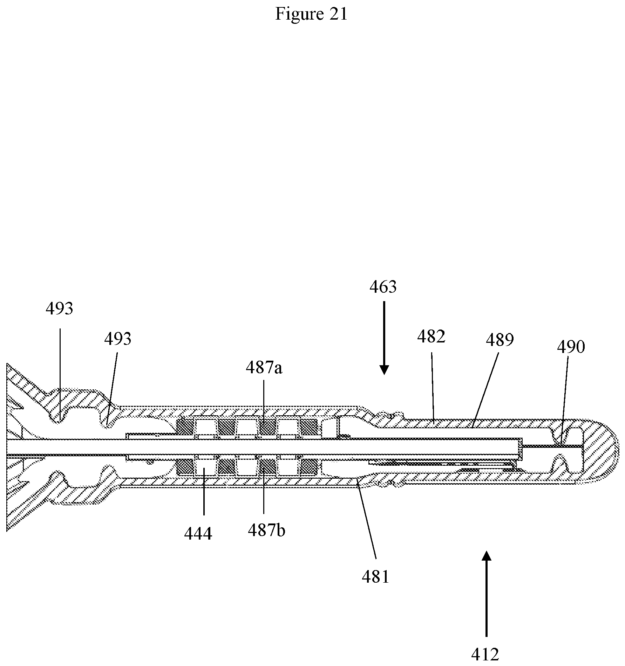

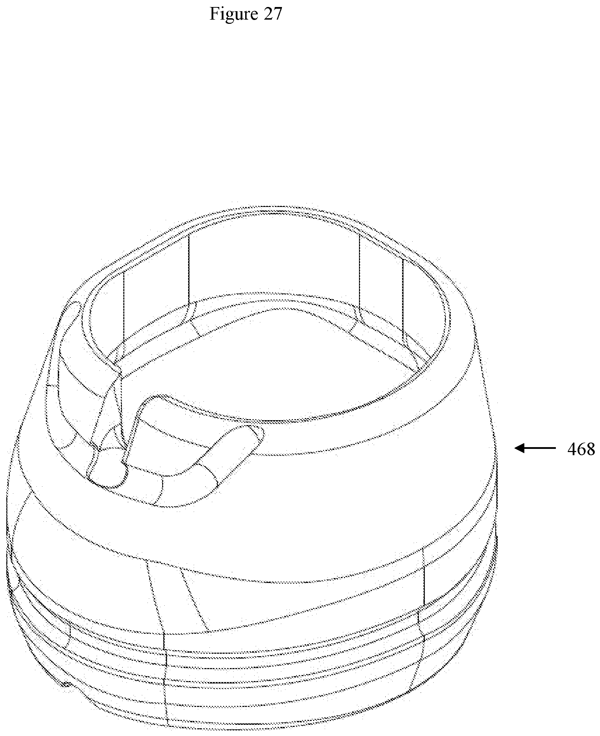

Figures 17 to 31 depict a third manometer 410**.** Its overall construction can best be understood with reference to Figure 19. Body 418 is in this embodiment formed by body halves 418a, 418b and body end cap 428, all of them received in and secured by a cup-shaped outer body part 418c. The body 418 houses circuit board 438 carrying battery 416 and pressure transducers 440, which as before include transducers 440' sensitive in use to pressure in the anal sphincter and a pressure sensor 440" disposed nearer to the distal end of probe 412 to sense pressure in the rectum, or in a balloon inflated in the rectum.

In this embodiment, the probe 412 comprises (a) a probe assembly 481 comprising a narrowed finger 442 of circuit board 438 on which the transducers 440 are carried and (b) a cover 482 comprising a stem portion 483 around the finger 442, and a bell-mouth portion 484 which engages with the body 418. The cover 482 comprises an elastomeric material in the present embodiment and is manufactured by overmoulding upon the body 418 and the probe assembly 481. That is, the probe assembly 481 and a domed end part 485 of the body 418 are placed in a suitable mould and the elastomer is injected around it, within the mould, to form the cover 482. Intimate engagement of the cover 482 with the assembly within it is thereby assured.

To define the pressure cells within the overmoulded cover 482, pre-formed cell parts 486, 487 are placed upon opposite sides of the circuit board 438 prior to the overmoulding process. Each cell part 486, 487 defines multiple pressure cells 444 each overlying a respective transducer 440. The pressure cells 444 are thus not filled with resin during the overmoulding process, but remain air-filled (or filled with another gas or pressure medium) to transmit pressure to the transducer 440 within, as explained previously. The cell parts 486, 487 may comprise the same elastomer material as the cover 482 into which they are integrated, so that this structure behaves as a unitary component.

In the present embodiment, a grid structure 487a, 487b is placed around the pressure cells 444, being interdigitated with them. The grid structure comprises a stiffer material than the elastomer of the cell parts 486, 487 and serves to prevent walls of the pressure cells 444 from bowing outward under pressure, thereby helping to ensure that pressure applied to the probe 412 produces a measurable change in pressure in the cell 444. However, in other embodiments the grid structure 487 is dispensed with, and the material of the cover 482 between the pressure cells 444 provides sufficient stiffness for this purpose.

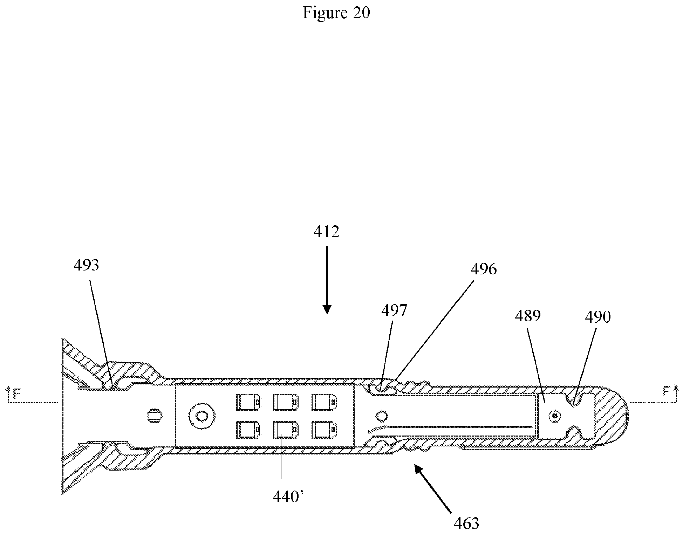

If the cover 482 moves relative to the assembly within it, this may cause unwanted deformation of the pressure cells 444 leading to spurious pressure signals or loss of sensor calibration. Such movement could, if measures were not taken to prevent it, be caused by application of the collar of the sheath, or by pressure exerted on the probe 412 during testing. To prevent it, interlocks are provided on the probe assembly 481 and on the domed part 485 of the body 418. On its distal end region, the finger 442 carries a pair of interlock parts 489a, 489b which are screwed to one another, sandwiching the finger 442 between them, and which carry interlock features which can best be seen in Figures 20 through 24, being formed as recesses 490, 491 which receive the material of the cover 482 during the overmoulding process and which serve to mechanically lock the cover 482 against both axial and rotational movement relative to the probe assembly 481 within.

Similarly, the domed part 485 of the body 418 carries interlock features in the form of trenches 492 and recesses 493 to receive the material of the cover 482 and to interlock with it. The shape of the interlock features 490, 491, 492, 493 may differ in other embodiments.

In Figures 17 and 19, reference 494 denotes a record button disposed in groove 426 to be easily actuated by the user's thumb. Beneath it is a switch 494a actuable through the flexible outer skin of the manometer. It can be used to initiate recordal of data. It is also used by the clinician to give control inputs to an associated processing device (to be discussed further below). Thus, for example, the clinician can use the record button 494 to step through options or test protocols on the processing device, without touching the processing device itself. This is desirable as it prevents transfer of pathogens to the processing device from the hands of the clinician, during a procedure.

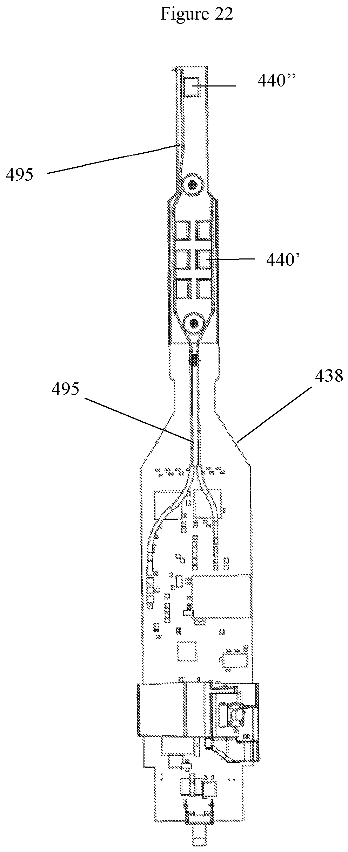

In this embodiment, the probe 412 is provided with a heating arrangement. Prior to use, the probe 412 will typically be exposed to room temperature of something of the order of 21 Celsius. When inserted in the patient, it experiences a higher temperature of the order of 37 Celsius. The pressure in the cells 444 varies with temperature due to thermal expansion, especially of the pressure medium in the cells, which may be air. If the probe temperature varies during use then this will cause variation of the measured pressures, undesirably distorting the data obtained. This could at least to some degree be compensated for by measuring probe temperature and adjusting sensor outputs accordingly in software, but the probe 412 cannot be expected to heat uniformly so that such compensation must be expected to be imperfect. Instead, in the present embodiment the heating arrangement is used to pre-warm the probe 412 to approximate body temperature, so that little or no subsequent temperature change takes place during testing. The heating arrangement may take any suitable form. In the illustrated example it comprises a resistive heater wire 495 carried on the circuit board 438 and extending along the length of its finger 442 - see Figure 22 in particular. A signal applied to the heater wire is controlled using a heat sensor to regulate probe temperature to approximate body temperature.

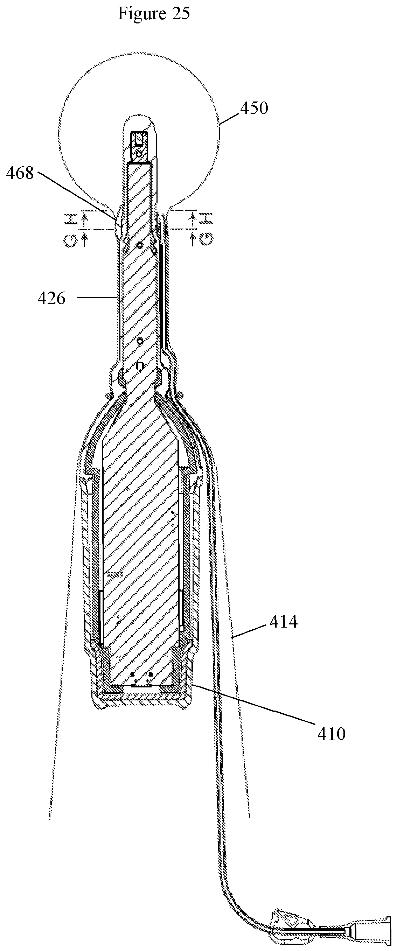

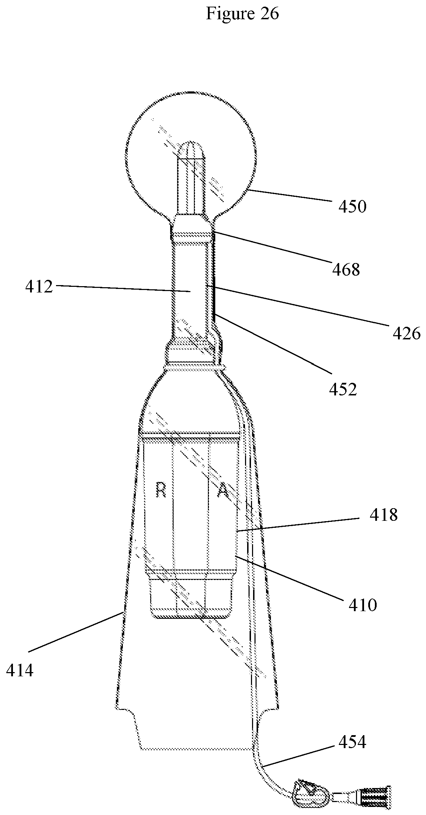

As depicted in Figures 25 and 26, the manometer 410 is configured to receive and operate with a disposable sheath 414, which covers the manometer to prevent transfer of infection and which also provides a balloon portion 450 to be inflated within the rectum in certain tests. The sheath 414 comprises a flexible impermeable skin shaped as a sleeve with a stem portion 452 to cover the probe 412 and an enlarged portion to cover the body 418. The stem portion 452 terminates in the balloon 450 which is inflatable through a tube 454. Part-way along its length the stem portion 452 is provided with a collar 468.

As depicted in Figure 17, cover 482 of the probe 412 comprises a collar-receiving feature 463 which in the present embodiment comprises (a) a circular seal feature 496 and (b) a non-circular location feature 497. The seal feature 496 has a double-convex profile and the location feature 497 forms a shoulder leading from a proximal probe portion 464b to a narrower tip portion 464a of the probe 412.

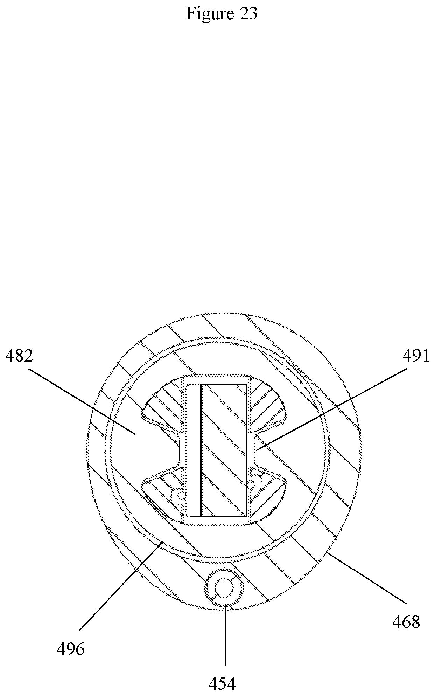

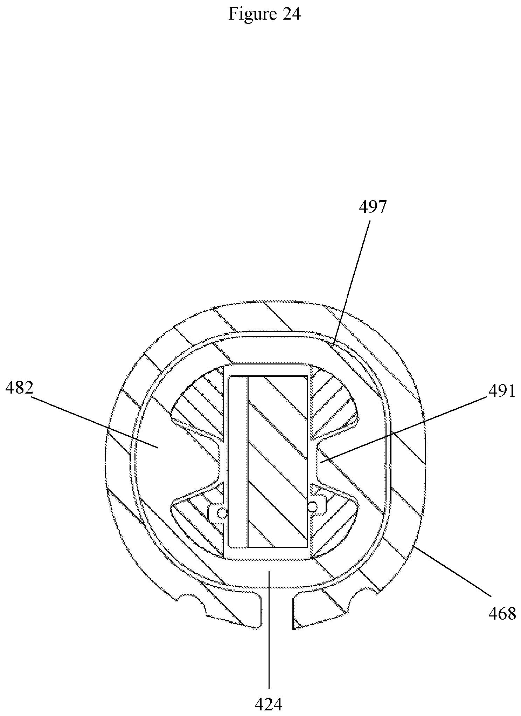

The collar 468 is shaped to seat upon and seal against the collar-receiving formation 463, as in Figures 23 to 26. Note from Figure 23 that the circular seal feature 496 of the probe cover 482 engages with a circular internal region of the collar 468, forming a seal therewith which is not impaired if there is some slight rotation of one relative to the other. But in Figure 24 it can be seen that the location portion 497 of the probe cover 424 is non-circular, so that the complementarily-shaped region of the interior of the collar 468 which engages this part can only be received in the correct angular position. This ensures that tube 454 of the sheath 450 aligns with groove 426 in the exterior of the probe cover 482, whose function is to receive the tube and prevent it being crushed by applied pressure.

As in the previous embodiment, the combination of the collar 468 with the collar-receiving formation 463 forms a seal against deflation of the balloon 450. It also locates the sheath 414 and its balloon 450 at the required place along the length of the probe 412, with the distal probe portion 464a in the balloon 450.

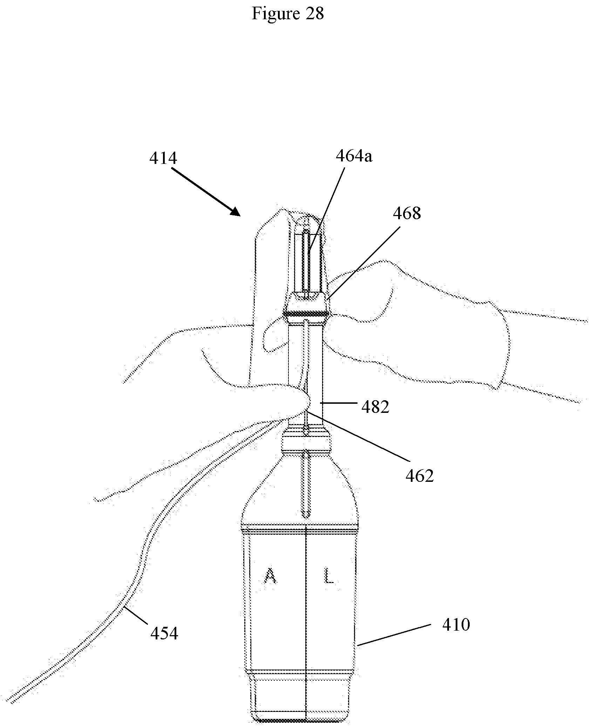

Figure 28 serves to illustrate the process of applying the sheath 414. It is supplied "inside out", with the collar 468 conveniently exposed so that it can be fitted to the distal probe portion 464a. Because the collar 468 can only be received on the collar-receiving formation 463 in the correct orientation (rotational position) it ensures that the tube 454 aligns with longitudinal recess 462 formed in the probe cover 482. The tube 454 can thus easily be laid along the recess 462. The sheath 414 is then drawn down over the manometer 410
Note that in all of the above-described embodiments, the manometer 10, 210, 410 is able to concurrently and independently sense (a) pressures applied to a proximal portion of the probe 12, 212, 312 and (b) pressure applied to the tip portion of the probe by pressure medium in the balloon portion 50, 250, 350, 450 of the sheath 14, 214, 314, 414.

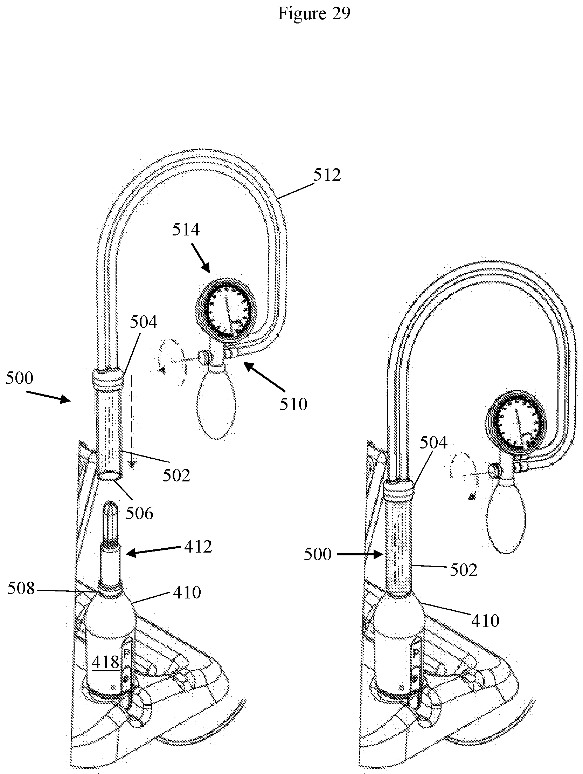

Figure 29 illustrates a process for testing and in some cases calibrating the manometer 410 using a testing sleeve 500 having a tubular body 502 closed by an end cap 504 and serving as a pressure chamber. In use, the probe 412 is inserted into the testing sleeve 500 until mouth 506 of the testing sleeve seats upon and seals with a shoulder 508 on the distal end of the body 418. The effect is to create a pressure chamber containing the probe 412 which is able to be pressurised to test the manometer's pressure response. In the illustrated example a pump 510 is connected through flexible air lines 512 to the end cap 504 and so to the pressure chamber, a pressure sensor 514 being used to determine the applied pressure. Known pressures can thus be exerted on the probe 412, and the responses of its pressure transducers can be determined, to establish whether they are an accurate representation of the applied pressure.

Output signals from the pressure transducers 40, 240, 440 may in certain embodiments be passed to their point of use through a physical connection such as electrical wires. But in the present embodiment the transducer data is instead transmitted wirelessly. The term "wireless" as used herein should be understood to include any suitable mode of data transfer which does not use a corporeal connection from transmitter to receiver, but a radio connection is used in the present embodiment. The widely known *Bluetooth*^{®} standard could be used for the purpose, but due to concerns over latency of communications by this method a different form of wireless link has been adopted in the present embodiment. It uses *WiFi*^{®}*.* A suitable transceiver and aerial are carried on the circuit board assembly 38.

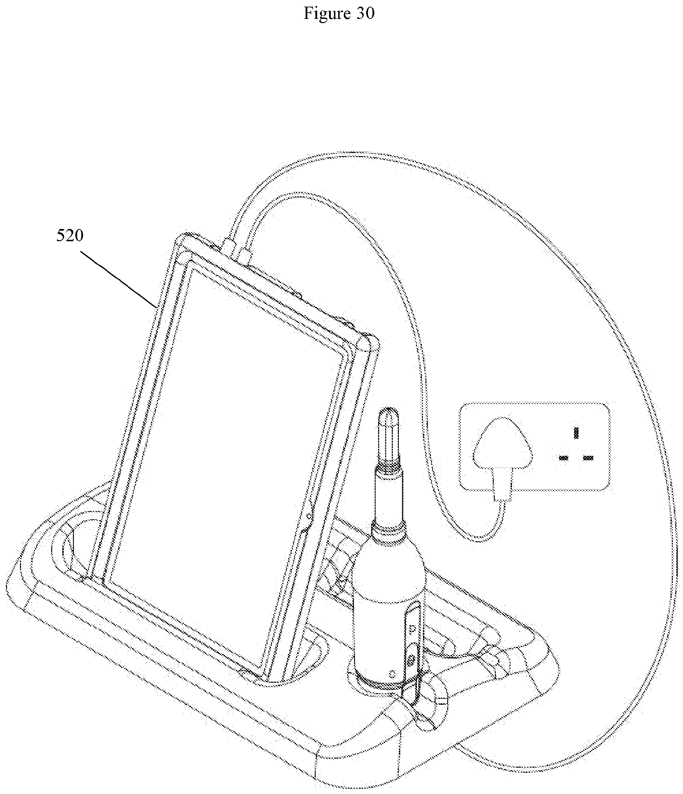

A digital processing device 520 (see Figure 30) is used in the present embodiment to receive the transducer data, to view it live and/or to store it, and to present it in suitable form to user(s). This may take the form of a computer, typically at the time of writing expected to be a tablet computer, running a suitable application. For avoidance of cross-contamination, a single-use pack provided to the user may include, in addition to the disposable sheath 14, 214, 314, 414, a disposable cover for the processing device. The pack may further include disposable gloves, disinfectant wipes and a sachet of lubricant. The disposable cover may, after use, be used to contain the other consumables ready for disposal.

The digital processing device receives the wirelessly transmitted data from the manometer 10, 210, 410. It may do so through one of its on-board wireless interfaces, or it may be connected to an external transceiver through one of its I/O connectors.

Procedures for anorectal manometry are well known to clinicians. It is not intended herein to provide a detailed description of such procedures. Certain aspects of the use of the manometer made possible by aspects of the present disclosure will be described. The manometer 10, 210, 410 may be used in clinical testing but it may also be used in remedial procedures, such as training through exercises involving provision of biofeedback.

Once communication between the manometer 10, 210, 410 and the digital processing device is established, the testing sleeve 500 can be used to verify pressure sensor function. Sensor output data - at this and subsequent stages - may be provided in real time to the clinician or other user through the wireless interface and the processing device. The application running on the processing device may display real time or recorded data through graphics such as graphs.

The probe 12, 212, 412 is typically to be inserted through the anal passage into the rectum. It may not always be clear through observation alone whether the probe 12, 212, 412 has been fully inserted, especially in relation to obese patients. When the distal portion of the probe 12, 212, 412 emerges through the anal passage into the rectum, pressure acting upon this portion of the probe 12, 212, 412 normally decreases. This local decrease of pressure can be detected through the rectal pressure cell 44", 444". Hence a decrease in pressure on this cell, in conjunction with sustained pressure on other pressure cells, can be interpreted as indicative of proper insertion of the probe.

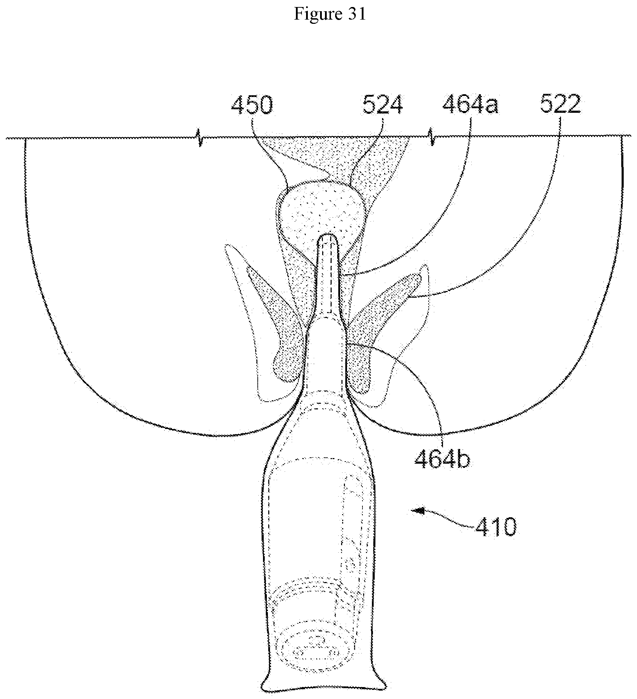

Figure 31 depicts the manometer 410 in use. Its proximal portion 464b is seen to be within the anal sphincter 522 so that pressure cells of the proximal portion 464b directly measure anal pressures. The distal tip portion 464a is seen to extend into the rectum 524. The balloon portion 450 is here inflated, so that the rectal sensor is exposed to the pressure in the balloon portion 450.

Pressure outputs derived from individual pressure cells 444 may be displayed individually to the user, or outputs forming groups of cells may be aggregated, e.g. by averaging. In one embodiment, concurrent displays presented on the digital processing device respectively show (1) a graph of the averaged value of all the pressure cells 444 in the sphincter region; (2) multiple graphs of values averaged over all the sensors at a given position along the probe's length (to give an idea of how pressure is varying through the length of the anal passage); and (c) individual graphs of pressures measured by each cell.

Responses to instructions given by the clinician (such as an instruction to clench, or to cough) can be observed in real time, as can responses to rectal stimulation by means of the balloon 450.

The facility, offered by the manometers 10, 210, 410, to simultaneously measure (a) pressure applied to the proximal portion of the probe 12, 212, 312, 212 by the anal sphincter and (b) pressure in the balloon portion 50, 250, 450 is useful in a range of clinical tests.

It is emphasised again that while embodiments have been described in detail herein for purposes of enablement and explanation, it is understood that the invention is solely defined by the scope of the appended claims.

## Claims

1. A sheath (14, 214, 314, 414) for use with an anorectal manometer (10, 210, 410) of the type comprising a probe (12, 212, 412) comprising a proximal portion (264b) and a distal tip portion (264a, 464a), the probe (12, 212, 412) being insertable through an anus so that the distal tip portion (264a, 464a) extends into a rectum, and the probe (12, 212, 412) being provided with a plurality of proximal pressure sensors (40, 164, 240) responsive to pressures applied to the proximal portion (264b) of the probe (12, 212, 412),
the sheath (14, 214, 314, 414) being formed to removably receive the probe (12, 212, 412) and to provide a continuous covering extending from the proximal portion (264b) to the distal tip portion (264a, 464a) of the probe (12, 212, 412), and the sheath (14, 214, 314, 414) comprising a proximal stem portion (52) for receiving at least the proximal portion (264b) of the probe (12, 212, 412), a distal balloon portion (50, 50a, 250, 350, 450), and an inflation conduit (54, 254, 354, 454) communicating with the balloon portion's (50, 50a, 250, 350, 450) interior, enabling inflation of the balloon portion (50, 50a, 250, 350, 450) with a pressure medium introduced through the inflation conduit (54, 254, 354, 454) whilst the balloon portion (50, 50a, 250, 350, 450) is disposed in the rectum,
wherein the sheath (14, 214, 314, 414) comprises flexible membranous material so that pressure applied to the stem portion of the sheath (14, 214, 314, 414) is transmitted through the sheath (14, 214, 314, 414) to the proximal portion (264b) of the probe (12, 212, 412), to be sensed by the pressure sensors, and
wherein the sheath (14, 214, 314, 414) comprises means for forming a seal against flow of the pressure medium from the balloon portion (50, 50a, 250, 350, 450) into the stem portion of the sheath (14, 214, 314, 414).

2. A sheath (14, 214, 314, 414) as claimed in claim 1 in which the means for forming a seal comprises a collar (268, 368, 468) having a through-going opening configured to receive the tip portion (264a, 464a) of the probe (12, 212, 412) so that the distal portion (264a, 464a) of the probe (12, 212, 412) extends through the collar (268, 368, 468) into the balloon portion (50, 50a, 250, 350, 450) of the sheath (14, 214, 314, 414).

3. A sheath (14, 214, 314, 414) as claimed in claim 2 in which the sheath (14, 214, 314, 414) comprises a closure comprising a flexible skin which forms a seal over the through-going opening of the collar (268, 368, 468), and which is able to receive the distal tip portion (264a, 464a) of the probe (12, 212, 412), so that the distal tip portion (264a, 464a) of the probe (12, 212, 412) is able to extend into the balloon portion (50, 50a, 250, 350, 450).

4. . A sheath (14, 214, 314 ,414) as claimed in claim 2 in which the collar (268, 368, 468) is disposed within an envelope formed by the flexible membranous material of the sheath (14, 214, 314, 414), its periphery being sealingly secured thereto.

5. An assembly for anorectal manometry comprising a sheath as claimed in any preceding claim and a manometer (10, 210, 410) comprising a probe (12, 212, 412) comprising a proximal portion (264b) and a distal tip portion (264a, 464a), the probe (12, 212, 412) being insertable through an anus so that the distal tip portion extends into a rectum, and the probe being provided with a plurality of proximal pressure sensors (40, 164, 240) responsive to pressures applied to the proximal portion (264b) of the probe (12, 212, 412).

6. An assembly as claimed in claim 5 in which the distal tip portion (264a, 464a) of the probe (12, 212, 412) is provided with a rectal pressure sensor (44") which is responsive to pressure applied to the distal tip portion (264a, 464a) of the probe (12, 212, 412) and which is thus able to detect rectal pressure when the balloon portion (50, 50a, 250, 350, 450) is not inflated, and to detect pressure of the pressure medium in the balloon portion (50, 50a, 250, 350, 450) when the balloon portion (50, 50a, 250, 350, 450) is inflated.

7. An assembly as claimed in claim 5 or claim 6 in which the collar (268, 368, 468) is configured to form a seal against the tip portion (264a, 464a) of the probe (12, 212, 412), thereby preventing flow from the balloon portion (50, 50a, 250, 350, 450) through the collar's (268, 368, 468) through-going opening into the stem portion (52) of the sheath (14, 214, 314, 414), in use.

8. An assembly as claimed in any of claims 5 to 7 in which the probe (12, 212, 412) comprises a location feature (263) between the proximal portion (264b) and the distal tip portion (264a, 464a), so that the tip portion (264a, 464a) of the probe (12, 212, 412) is able to be advanced through the collar (268, 368, 468) until the collar (268, 368, 468) engages the registration feature, blocking further advancement of the probe (12, 212, 412) through the collar (268, 368, 468).

9. An assembly as claimed in claim 8 in which the registration feature comprises a shoulder on the probe (12, 212, 412).

10. An assembly as claimed in any of claims 5 to 9 in which the inflation conduit (54, 254, 354, 454) comprises a tube communicating with the balloon portion (50, 50a, 250, 350, 450) of the sheath (14, 214, 314, 414), and the probe (12, 212, 412) has a recess extending lengthwise along at least part of the probe (12, 212, 412) to receive the tube.

11. An assembly as claimed in claim 10 when dependent on claim 2 in which the tube is coupled to the collar (268, 368, 468).

12. An assembly as claimed in claim 11 in which at least a portion of the probe (12, 212, 412) has a non-circular external profile and at least part of the through-going opening of the collar (268, 368, 468) has a complementary non-circular internal profile, so that the collar (268, 368, 468) is receivable on the probe's (12, 212, 412) tip portion (264a, 464a) only in a limited number of rotational positions, in at least one of which the tube is aligned to be received in the recess.

13. An assembly as claimed in claim 11 or claim 12 in which at least a portion of the probe (12, 212, 412) has a circular external profile and at least part of the through-going opening of the collar (268, 368, 468) has a complementary circular internal profile, so that the probe (12, 212, 412) and the collar (268, 368, 468) together provide a seal against egress of gas from the balloon portion (50, 50a, 250, 350, 450) of the sheath (14, 214, 314, 414).

14. An assembly as claimed in any of claims 5 to 13 in which the manometer (10, 210, 410) further comprises a body from which the probe (12, 212, 412) projects and using which the manometer (10, 210, 410) is able to be held by the hand of an operative, in use, the sheath (14, 214, 314, 414) comprising an enlarged proximal portion (264b) to cover the body in use.

15. An assembly as claimed in any of claims 5 to 14 in which the proximal pressure sensors (40, 164, 240) are solid-state sensors housed in the probe (12, 212, 412).

## Patentansprüche

1. Hülle (14, 214, 314, 414) zur Verwendung mit einem anorektalen Manometer (10, 210, 410) des Typs, der eine Sonde (12, 212, 412) umfasst, die einen proximalen Abschnitt (264b) und einen distalen Spitzenabschnitt (264a, 464a) umfasst, wobei die Sonde (12, 212, 412) durch einen Anus einführbar ist, sodass sich der distale Spitzenabschnitt (264a, 464a) in ein Rektum erstreckt, und wobei die Sonde (12, 212, 412) mit einer Vielzahl von proximalen Drucksensoren (40, 164, 240) bereitgestellt wird, die auf Drücke ansprechen, die auf den proximalen Abschnitt (264b) der Sonde (12, 212,412) ausgeübt werden,
wobei die Hülle (14, 214, 314, 414) so ausgebildet ist, dass sie die Sonde (12, 212, 412) entfernbar aufnimmt und eine durchgehende Abdeckung bereitstellt, die sich von dem proximalen Abschnitt (264b) zu dem distalen Spitzenabschnitt (264a, 464a) der Sonde (12, 212, 412) erstreckt, und die Hülle (14, 214, 314, 414) einen proximalen Schaftabschnitt (52) zur Aufnahme mindestens des proximalen Abschnitts (264b) der Sonde (12, 212, 412), einen distalen Ballonabschnitt (50, 50a, 250, 350, 450) und eine Aufblasleitung (54, 254, 354, 454) umfasst, die mit dem Inneren des Ballonabschnitts (50, 50a, 250, 350, 450) in Verbindung steht, was das Aufblasen des Ballonabschnitts (50, 50a, 250, 350, 450) mit einem Druckmedium ermöglicht, das durch die Aufblasleitung (54, 254, 354, 454) eingeführt wird, während der Ballonabschnitt (50, 50a, 250, 350, 450) im Rektum angeordnet ist,
wobei die Hülle (14, 214, 314, 414) flexibles Membranmaterial umfasst, sodass auf den Schaftabschnitt der Hülle (14, 214, 314, 414) ausgeübter Druck durch die Hülle (14, 214, 314, 414) auf den proximalen Abschnitt (264b) der Sonde (12, 212, 412) übertragen wird, um von den Drucksensoren erfasst zu werden, und
wobei die Hülle (14, 214, 314, 414) Mittel zum Bilden einer Dichtung gegen ein Strömen des Druckmediums aus dem Ballonabschnitt (50, 50a, 250, 350, 450) in den Schaftabschnitt der Hülle (14, 214, 314, 414) umfasst.

2. Hülle (14, 214, 314, 414) nach Anspruch 1, wobei das Mittel zum Bilden einer Dichtung einen Kragen (268, 368, 468) mit einer durchgehenden Öffnung umfasst, die konfiguriert ist, um den Spitzenabschnitt (264a, 464a) der Sonde (12, 212, 412) aufzunehmen, sodass sich der distale Abschnitt (264a, 464a) der Sonde (12, 212, 412) durch den Kragen (268, 368, 468) in den Ballonabschnitt (50, 50a, 250, 350, 450) der Hülle (14, 214, 314, 414) erstreckt.

3. Hülle (14, 214, 314, 414) nach Anspruch 2, wobei die Hülle (14, 214, 314, 414) einen Verschluss umfasst, der eine flexible Haut umfasst, die eine Dichtung über der durchgehenden Öffnung des Kragens (268, 368, 468) bildet und die in der Lage ist, den distalen Spitzenabschnitt (264a, 464a) der Sonde (12, 212, 412) aufzunehmen, sodass der distale Spitzenabschnitt (264a, 464a) der Sonde (12, 212, 412) in der Lage ist, sich in den Ballonabschnitt (50, 50a, 250, 350, 450) zu erstrecken.

4. Hülle (14, 214, 314,414) nach Anspruch 2, wobei der Kragen (268, 368, 468) innerhalb einer Umhüllung angeordnet ist, die durch das flexible membranartige Material der Hülle (14, 214, 314, 414) gebildet wird, wobei sein Umfang abdichtend daran befestigt ist.

5. Baugruppe für die anorektale Manometrie, umfassend eine Hülle nach einem der vorstehenden Ansprüche und einen Manometer (10, 210, 410), umfassend eine Sonde (12, 212, 412), umfassend einen proximalen Abschnitt (264b) und einen distalen Spitzenabschnitt (264a, 464a), wobei die Sonde (12, 212, 412) durch einen Anus einführbar ist, sodass sich der distale Spitzenabschnitt in ein Rektum erstreckt, und wobei die Sonde mit einer Vielzahl von proximalen Drucksensoren (40, 164, 240) bereitgestellt wird, die auf Drücke ansprechen, die auf den proximalen Abschnitt (264b) der Sonde (12, 212, 412) ausgeübt werden.

6. Baugruppe nach Anspruch 5, wobei der distale Spitzenabschnitt (264a, 464a) der Sonde (12, 212, 412) mit einem rektalen Drucksensor (44") bereitgestellt wird, der auf Druck anspricht, der auf den distalen Spitzenabschnitt (264a, 464a) der Sonde (12, 212, 412) ausgeübt wird, und der somit in der Lage ist, den rektalen Druck zu erfassen, wenn der Ballonabschnitt (50, 50a, 250, 350, 450) nicht aufgeblasen ist, und den Druck des Druckmediums im Ballonabschnitt (50, 50a, 250, 350, 450) zu erfassen, wenn der Ballonabschnitt (50, 50a, 250, 350, 450) aufgeblasen ist.

7. Baugruppe nach Anspruch 5 oder 6, wobei der Kragen (268, 368, 468) konfiguriert ist, um eine Dichtung gegen den Spitzenabschnitt (264a, 464a) der Sonde (12, 212, 412) zu bilden, wodurch im Gebrauch eine Strömung von dem Ballonabschnitt (50, 50a, 250, 350, 450) durch die durchgehende Öffnung des Kragens (268, 368, 468) in den Schaftabschnitt (52) der Hülle (14, 214, 314, 414) verhindert wird.

8. Baugruppe nach einem der Ansprüche 5 bis 7, wobei die Sonde (12, 212, 412) ein Positionierungsmerkmal (263) zwischen dem proximalen Abschnitt (264b) und dem distalen Spitzenabschnitt (264a, 464a) umfasst, sodass der Spitzenabschnitt (264a, 464a) der Sonde (12, 212, 412) durch den Kragen (268, 368, 468) vorgeschoben werden kann, bis der Kragen (268, 368, 468) mit dem Ausrichtungsmerkmal in Eingriff kommt, was ein weiteres Vorschieben der Sonde (12, 212, 412) durch den Kragen (268, 368, 468) blockiert.

9. Baugruppe nach Anspruch 8, wobei das Ausrichtungsmerkmal eine Schulter an der Sonde (12, 212, 412) umfasst.

10. Baugruppe nach einem der Ansprüche 5 bis 9, wobei die Aufblasleitung (54, 254, 354, 454) ein Rohr umfasst, das mit dem Ballonabschnitt (50, 50a, 250, 350, 450) der Hülle (14, 214, 314, 414) in Verbindung steht, und die Sonde (12, 212, 412) eine Aussparung aufweist, die sich in Längsrichtung entlang mindestens eines Teils der Sonde (12, 212, 412) erstreckt, um das Rohr aufzunehmen.

11. Baugruppe nach Anspruch 10, wenn abhängig von Anspruch 2, wobei das Rohr an den Kragen (268, 368, 468) gekoppelt ist.

12. Baugruppe nach Anspruch 11, bei der mindestens ein Abschnitt der Sonde (12, 212, 412) ein nicht kreisförmiges äußeres Profil aufweist und mindestens ein Teil der durchgehenden Öffnung des Kragens (268, 368, 468) ein komplementäres nicht kreisförmiges inneres Profil aufweist, sodass der Kragen (268, 368, 468) auf dem Spitzenabschnitt (264a, 464a) der Sonde (12, 212, 412) nur in einer begrenzten Anzahl von Drehpositionen aufnehmbar ist, wobei in mindestens einer davon das Rohr so ausgerichtet ist, um in der Aussparung aufgenommen zu werden.

13. Baugruppe nach Anspruch 11 oder 12, wobei mindestens ein Abschnitt der Sonde (12, 212, 412) ein kreisförmiges äußeres Profil aufweist und mindestens ein Teil der durchgehenden Öffnung des Kragens (268, 368, 468) ein komplementäres kreisförmiges inneres Profil aufweist, sodass die Sonde (12, 212, 412) und der Kragen (268, 368, 468) zusammen eine Dichtung gegen den Austritt von Gas aus dem Ballonabschnitt (50, 50a, 250, 350, 450) der Hülle (14, 214, 314, 414) bereitstellen.

14. Baugruppe nach einem der Ansprüche 5 bis 13, wobei das Manometer (10, 210, 410) ferner einen Körper umfasst, von dem die Sonde (12, 212, 412) vorsteht und mit dem das Manometer (10, 210, 410) im Gebrauch von der Hand eines Bedieners gehalten werden kann, wobei die Hülle (14, 214, 314, 414) einen vergrößerten proximalen Abschnitt (264b) umfasst, um den Körper im Gebrauch abzudecken.

15. Baugruppe nach einem der Ansprüche 5 bis 14, wobei die proximalen Drucksensoren (40, 164, 240) in der Sonde (12, 212, 412) untergebrachte Festkörpersensoren sind.

## Revendications

1. Gaine (14, 214, 314, 414) destinée à être utilisée avec un manomètre anorectal (10, 210, 410) du type comprenant une sonde (12, 212, 412) comprenant une portion proximale (264b) et une portion pointe distale (264a, 464a), la sonde (12, 212, 412) pouvant être insérée à travers un anus de sorte que la portion pointe distale (264a, 464a) s'étend dans un rectum, et la sonde (12, 212, 412) étant pourvue d'une pluralité de capteurs de pression proximaux (40, 164, 240) sensibles à des pressions appliquées à la portion proximale (264b) de la sonde (12, 212, 412),
la gaine (14, 214, 314, 414) étant formée pour recevoir de manière amovible la sonde (12, 212, 412) et pour fournir un revêtement continu s'étendant de la portion proximale (264b) à la portion pointe distale (264a, 464a) de la sonde (12, 212, 412), et la gaine (14, 214, 314, 414) comprenant une portion tige proximale (52) pour recevoir au moins la portion proximale (264b) de la sonde (12, 212, 412), une portion ballonnet (50, 50a, 250, 350, 450) distale, et un conduit de gonflage (54, 254, 354, 454) communiquant avec l'intérieur de la portion ballonnet (50, 50a, 250, 350, 450), permettant un gonflage de la portion ballonnet (50, 50a, 250, 350, 450) avec un milieu sous pression introduit à travers le conduit de gonflage (54, 254, 354, 454) pendant que la portion ballonnet (50, 50a, 250, 350, 450) est disposée dans le rectum,
dans laquelle la gaine (14, 214, 314, 414) comprend un matériau membraneux flexible de sorte que la pression appliquée à la portion tige de la gaine (14, 214, 314, 414) est transmise à travers la gaine (14, 214, 314, 414) vers la portion proximale (264b) de la sonde (12, 212, 412), pour être détectée par les capteurs de pression, et
dans laquelle la gaine (14, 214, 314, 414) comprend un moyen pour former un joint d'étanchéité contre un écoulement du milieu sous pression depuis la portion ballonnet (50, 50a, 250, 350, 450) dans la portion tige de la gaine (14, 214, 314, 414).

2. Gaine (14, 214, 314, 414) selon la revendication 1, dans laquelle le moyen de formation d'un joint d'étanchéité comprend un collier (268, 368, 468) présentant une ouverture traversante conçue pour recevoir la portion pointe (264a, 464a) de la sonde (12, 212, 412) de sorte que la portion distale (264a, 464a) de la sonde (12, 212, 412) s'étend à travers le collier (268, 368, 468) jusque dans la portion ballonnet (50, 50a, 250, 350, 450) de la gaine (14, 214, 314, 414).

3. Gaine (14, 214, 314, 414) selon la revendication 2, dans laquelle la gaine (14, 214, 314, 414) comprend une fermeture comprenant un habillage flexible qui forme un joint d'étanchéité sur l'ouverture traversante du collier (268, 368, 468), et qui est apte à recevoir la portion pointe distale (264a, 464a) de la sonde (12, 212, 412), de sorte que la portion pointe distale (264a, 464a) de la sonde (12, 212, 412) est apte à s'étendre dans la portion ballonnet (50, 50a, 250, 350, 450).

4. Gaine (14, 214, 314, 414) selon la revendication 2, dans laquelle le collier (268, 368, 468) est disposé à l'intérieur d'une enveloppe formée par le matériau membraneux flexible de la gaine (14, 214, 314, 414), sa périphérie y étant fixée de manière étanche.

5. Ensemble pour une manométrie anorectale comprenant une gaine selon l'une quelconque revendication précédente et un manomètre (10, 210, 410) comprenant une sonde (12, 212, 412) comprenant une portion proximale (264b) et une portion pointe distale (264a, 464a), la sonde (12, 212, 412) pouvant être insérée à travers un anus de sorte que la portion pointe distale s'étend dans un rectum, et la sonde étant pourvue d'une pluralité de capteurs de pression proximaux (40, 164, 240) sensibles à des pressions appliquées à la portion proximale (264b) de la sonde (12, 212, 412).

6. Ensemble selon la revendication 5, dans lequel la portion pointe distale (264a, 464a) de la sonde (12, 212, 412) est pourvue d'un capteur de pression rectale (44") qui est sensible à une pression appliquée à la portion pointe distale (264a, 464a) de la sonde (12, 212, 412) et qui est ainsi apte à détecter une pression rectale lorsque la portion ballonnet (50, 50a, 250, 350, 450) n'est pas gonflée, et à détecter une pression du milieu sous pression dans la portion ballonnet (50, 50a, 250, 350, 450) lorsque la portion ballonnet (50, 50a, 250, 350, 450) est gonflée.

7. Ensemble selon la revendication 5 ou la revendication 6, dans lequel le collier (268, 368, 468) est conçu pour former un joint d'étanchéité contre la portion pointe (264a, 464a) de la sonde (12, 212, 412), empêchant ainsi un écoulement depuis la portion ballonnet (50, 50a, 250, 350, 450) à travers l'ouverture traversante du collier (268, 368, 468) jusque dans la portion tige (52) de la gaine (14, 214, 314, 414), lors de l'utilisation.

8. Ensemble selon l'une quelconque des revendications 5 à 7, dans lequel la sonde (12, 212, 412) comprend un élément de positionnement (263) entre la portion proximale (264b) et la portion pointe distale (264a, 464a), de sorte que la portion pointe (264a, 464a) de la sonde (12, 212, 412) est apte à être avancée à travers le collier (268, 368, 468) jusqu'à ce que le collier (268, 368, 468) vienne en prise avec l'élément de repérage, bloquant tout avancement supplémentaire de la sonde (12, 212, 412) à travers le collier (268, 368, 468).

9. Ensemble selon la revendication 8, dans lequel l'élément de repérage comprend un épaulement sur la sonde (12, 212, 412).

10. Ensemble selon l'une quelconque des revendications 5 à 9, dans lequel le conduit de gonflage (54, 254, 354, 454) comprend un tube communiquant avec la portion ballonnet (50, 50a, 250, 350, 450) de la gaine (14, 214, 314, 414), et la sonde (12, 212, 412) présente un évidement s'étendant de manière longitudinale le long d'au moins une partie de la sonde (12, 212, 412) pour recevoir le tube.

11. Ensemble selon la revendication 10 lorsqu'elle dépend de la revendication 2, dans lequel le tube est accouplé au collier (268, 368, 468).

12. Ensemble selon la revendication 11, dans lequel au moins une portion de la sonde (12, 212, 412) présente un profil externe non circulaire et au moins une partie de l'ouverture traversante du collier (268, 368, 468) présente un profil interne non circulaire complémentaire, de sorte que le collier (268, 368, 468) peut être reçu sur la portion pointe (264a, 464a) de la sonde (12, 212, 412) uniquement dans un nombre limité de positions de rotation, dans au moins l'une parmi lesquelles le tube est aligné pour être reçu dans l'évidement.

13. Ensemble selon la revendication 11 ou la revendication 12, dans lequel au moins une portion de la sonde (12, 212, 412) présente un profil externe circulaire et au moins une partie de l'ouverture traversante du collier (268, 368, 468) présente un profil interne circulaire complémentaire, de sorte que la sonde (12, 212, 412) et le collier (268, 368, 468) assurent ensemble une étanchéité contre une sortie de gaz de la portion ballonnet (50, 50a, 250, 350, 450) de la gaine (14, 214, 314, 414).

14. Ensemble selon l'une quelconque des revendications 5 à 13, dans lequel le manomètre (10, 210, 410) comprend en outre un corps à partir duquel la sonde (12, 212, 412) fait saillie et à l'aide duquel le manomètre (10, 210, 410) est apte à être tenu par la main d'un opérateur, lors d'une utilisation, la gaine (14, 214, 314, 414) comprenant une portion proximale élargie (264b) pour recouvrir le corps lors d'une utilisation.

15. Ensemble selon l'une quelconque des revendications 5 à 14, dans lequel les capteurs de pression proximaux (40, 164, 240) sont des capteurs à semiconducteurs logés dans la sonde (12, 212, 412).
